# EUROPEAN PATENT SPECIFICATION

(11) **EP 4 498 077 B1**
(45) Date of publication and mention of the grant of the patent: **29.10.2025**
(21) Application number: 24188463.4
(22) Date of filing: 12.07.2024
(51) Int. Cl.: G01N 27/48, G01N 33/03

(54) **VOLTAMMETRIC METHOD FOR DETERMINING THYMOQUINONE**
VOLTAMMETRISCHE METHODE ZUR BESTIMMUNG VON THYMOQUINON
MÉTHODE VOLTAMÉTRIQUE POUR DÉTERMINER LA THYMOQUINONE

(30) Priority: 25.07.2023 PL 44567423; 11.12.2023 PL 44705623
(43) Date of publication of application: 29.01.2025
(73) Proprietor: Uniwersytet Lodzki, 90-136 Lodz (PL)
(72) Inventor: Smarzewska, Sylwia, 94-054 Lódz (PL); Koszelska, Kamila, 91-327 Lódz (PL); Swiderski, Michal, 96-100 Skierniewice (PL); Guziejewski, Dariusz, 87-851 Boniewo (PL); Skowron-Jaskólska, Monika, 94-048 Lódz (PL); Ullah, Nabi, 25000 Peshawar (PK); Miekos, Ewa, 91-134 Lódz (PL); Wypych, Monika, 25-437 Kielce (PL)
(74) Representative: Kicinska-Fujawa, Alicja

(56) References cited:
- MICHELITSCH ASTRID ET AL: "A simple differential pulse polarographic method for the determination of thymoquinone in black seed oil", vol. 14, no. 4, 1 July 2003 (2003-07-01), Hoboken, USA, pages 224 - 227, XP093223517, ISSN: 0958-0344, Retrieved from the Internet <URL:https://analyticalsciencejournals.onlinelibrary.wiley.com/doi/pdfdirect/10.1002/pca.707> DOI: 10.1002/pca.707
- RITA PETRUCCI ET AL: "Cyclic voltammetry, spectroelectrochemistry and electron spin resonance as combined tools to study thymoquinone in aprotic medium", ELECTROCHIMICA ACTA, ELSEVIER, AMSTERDAM, NL, vol. 60, 22 November 2011 (2011-11-22), pages 230 - 238, XP028353914, ISSN: 0013-4686, [retrieved on 20111122], DOI: 10.1016/J.ELECTACTA.2011.11.055
- KISHWAR FARAH: "Cyclic Voltammetric Studies of Thymoquinone with Iron (III)", vol. 56, no. 2, 6 March 2013 (2013-03-06), pages 59 - 69, XP093223402, Retrieved from the Internet <URL:https://typeset.io/pdf/cyclic-voltammetric-studies-of-thymoquinone-with-1ron-111-4v1l416u0z.pdf>

## Description

The subject matter of an invention is voltammetric method for determination of thymoquinone.

Tymoquinone is a strong phytochemical antioxidant. It has been proven that it effectively neutralizes ROS (reactive forms of oxygen), and thus can eliminate the adverse health effects resulting from their increased levels. Compounds with antioxidant properties therefore indirectly exhibit anticancer, hepatoprotective and also other properties. Therefore, research is currently being conducted on using such compounds as an alternative to substances used, for example, in chemotherapy, which may help reduce its harmful effects. Additionally, the potential effectiveness of thymoquinone in the treatment of allergic asthma, liver disease or diabetes has been proven. Therefore, it is extremely important to precisely determine thymoquinone in samples of various origins.

To date, several examples of the analytical determination of thymoquinone in various types of samples are known in the literature. Methods have been developed for the determination of this compound in plant extracts, black cumin seeds, oils pressed from seeds, but also in rat plasma and pharmaceutical preparations. The vast majority of developed procedures use chromatographic techniques, mainly high-performance liquid chromatography (HPLC) , but also thin-layer chromatography (TLC) and its variant - high-performance thin-layer chromatography (HPTLC) . The developed chromatographic procedures differ in the method of analyte detection, some use traditional UV-Vis detectors, others use more advanced MS, DAD or fluorescence detectors. In addition, they differ in the way samples are prepared for analysis, with most papers presenting the authors' sample preparation procedures.

Apart from the above-mentioned methods using chromatographic techniques, only one procedure based on electrochemical techniques is known in the literature. More specifically, it is about polarographic determination of thymoquinone using a mercury electrode (Michalitsch A, Rittmannsberger A: A Simple Differential Pulse Polarographic Method for the Determination of Thymoquinone in Black Seed Oil, Phytochem. Anal., 14, 224-227 (2003). The mentioned polarographic procedure is based on the use of the differential pulse polarography (DPP) technique. The authors of this publication prove the electrochemical activity of thymoquinone in a wide range of pH of the supporting electrolyte. They declared that they had tested several types of buffer in order to select the optimal composition of the supporting electrolyte for analyses. They test Britton-Robinson buffers, Sorensen buffers and acetate buffers (with different concentrations of H ^{+ ions} ), but always in the presence of methanol, and the volume ratio of methanol to the buffer solution is 7:3 (v/v). After selecting the optimal electrolyte, which was Sorensen's methanol buffer at pH 8.5 (7:3 v/v), a linear dependence of the thymoquinone peak current on the thymoquinone concentration was plotted, and on its basis the detection and quantification limits of the method were determined. In the final step, the authors of the publication describe the practical use of the developed procedure for direct determination of thymoquinone in oil samples.

In the state of the art there is no method for determining thymoquinone that would be universally applicable, i.e. suitable for determining the level of thymoquinone in samples of various origins.

The technical problem solved by the present invention is to provide an universal method for determining thymoquinone in samples of various origins, where thymoquinone is contained in a sample of a polar or non-polar nature, i.e. water or oil.

The subject of the invention is a method for determining thymoquinone using the square wave voltammetry (SWV) technique, characterized in that it includes steps where
- in the first step, equal volumes of acetic acid, boric acid and phosphoric acid are mixed at a concentration of 0.04 mol/L and adjusted to a pH in the range of 9.0-10.0, preparing a buffer solution which constitutes the supporting electrolyte (2);
- in the second step, a sample solution is prepared for analysis
   - for samples in which the medium (M) is oil - a solution of a food sample of the oil under test, is made by dissolving the oil in ethanol in a volumetric flask, then filled up to volume with ethanol to the mark with ethanol and stirred;
   - for samples in which the medium (M) is water or a water-soluble solvent - the sample solution is made by dissolving thymoquinone in water;
- in the third step, the electrochemical cell (1) is filled with the supporting electrolyte (2) and a magnetic stirrer (6), a platinum electrode (3) as an auxiliary electrode, a saturated silver chloride electrode (4) as a reference electrode and a working electrode (5) are introduced successively , where the electrode reaction of thymoquinone reduction takes place at the working electrode surface (5);
- in the fourth step, all three electrodes are connected to the potentiostat (7), each to a dedicated connection, and then the supporting electrolyte solution (2) is deoxidized for five minutes before measurement, using argon from the gas cylinder (8) constituting the gas source connected to electrochemical cell (1) with a supply tube (9), and the solution is mixed during deoxidation using a magnetic stirrer (6);
- in the fifth step, voltammograms are recorded with the determination of current reference values for standard solutions of known thymoquinone concentrations, whereby
   - for samples in which the medium (M) is oil, the recording of voltammograms includes steps where:
      a) voltammogram is recorded for pure supporting electrolyte, without any additives, constituting the blank;
      b) the voltammogram of the sample, prepared in the second step, is recorded and the peak current is measured at a potential of -165mV;
      c) a standard thymoquinone solution, which is a solution of thymoquinone in methanol, is added to the electrochemical cell (1) and the peak current is measured at a potential of -165 mV;
      d) step c) is repeated twice using additions of the standard thymoquinone solution containing the same amount of thymoquinone as in step c);
      e) from the peak current values obtained in points b)-d) the curve is plotted for *I*ₚ = *f* (X_{tym}), where
         *I*ₚ denotes peak current at a potential of -165 mV and *X*_{tym} denotes the amount of moles or grams of added thymoquinone;
      f) the point of intersection of the obtained curve of step e) with the horizontal axis is read, and based on the obtained *X*_{tym} , the content of thymoquinone in the electrochemical cell (1) and in the sample is calculated;
      g) then steps a)-f) are repeated three times and based on the results obtained, the average content of thymoquinone in the sample is calculated;
      , wherein all voltammograms are recorded in the potential range from -0.05V to -1.2V, with the SWV technique parameters being: amplitude 25 mV, step potential 4 mV and frequency 25 Hz;
   - for samples in which the medium (M) is water or a water-soluble solvent, recording of voltammograms includes steps where:
      h) voltammogram is recorded for pure supporting electrolyte, without any additives, constituting the blank;
      i) a standard thymoquinone solution, which is a solution of thymoquinone in water, is added to the electrochemical cell (1) and peak current is measured at a potential of -220 mV;
      j) then step i) is repeated minimum five times, recording voltammograms for the increasing concentration of thymoquinone in the electrochemical cell, which is created by the addition of the standard thymoquinone solution, and each time the peak current is measured at a potential of -220mV;
      k) steps h)-j) are repeated three times to obtain three measurement series;
      l) then the relationship *I*ₚ = *f*(c_{Tym}) is determined, where:
         *I*ₚ denotes averaged from three measurement series, the value of the peak current at a potential of -220mV for a given thymoquinone concentration,
         c_{Tym} - thymoquinone concentration in the electrochemical cell, and a equation of calibration curve is determined.
      m) then the voltammogram for the sample prepared in the second step is recorded and the peak current is measured at a potential of -220mV;
      n) the value of the peak current obtained in step m) is substituted into the equation from step l) as *I*ₚ, calculating the concentration of thymoquinone in the electrochemical cell and then the content in the sample;
      o) then steps m)-n) are repeated twice and, based on the results obtained, the average content of thymoquinone in the sample is calculated;
      , wherein all voltammograms are recorded in the potential range from 0.1V to -0.7V, with the SWV technique parameters being: amplitude 30mV, step potential 5mV and frequency 25Hz.

In the method according to the invention, the pH of the supporting electrolyte is adjusted to the appropriate pH using sodium or potassium hydroxide solutions.

In one of the variants of the method according to the invention, in the first step the pH of the supporting electrolyte is adjusted to pH equal to 9.0 with 0.2M sodium hydroxide, where in the second step the working electrode (5) is a diamond electrode doped with boron, with the medium (M) being water or a water-soluble solvent.

In another variant of the method according to the invention, in the first step the pH of the supporting electrolyte is adjusted to pH equal to 10.0 with 0.2M potassium hydroxide, where in the second step the working electrode (5) is an amalgam film electrode , with the medium (M) being oil.

In the method according to the invention, the electrode reaction constituting the basis of the electrochemical process carried out, consist of the reduction of the analyte, which takes place on a boron-doped diamond electrode or an amalgam film electrode. Both the working, reference and auxiliary electrodes are connected to the potentiostat, each to a dedicated connection.

The advantage of the method according to the invention is that enables direct determination of thymoquinone in aqueous solutions or in solutions based on water-soluble solvents or in oil samples after dissolving the sample in ethanol, without the need for preliminary sample preparation. The analysis is simple, very fast, and meets the principles of green chemistry, as measurements mainly use aqueous solutions, and organic solvents are used in an amount of no more than 0.5ml per single experiment. Additionally, the presented method according to the invention is based on the use of an electrolyte without toxic solvents. Moreover, as shown in the example carried out for a sample in which the medium (M) is water or a water-soluble solvent, the R2 coefficient obtained for the straight line is 0.9999, which guarantees excellent precision, accuracy and correctness of the measurement.

The subject of the invention is illustrated in the figures in which:

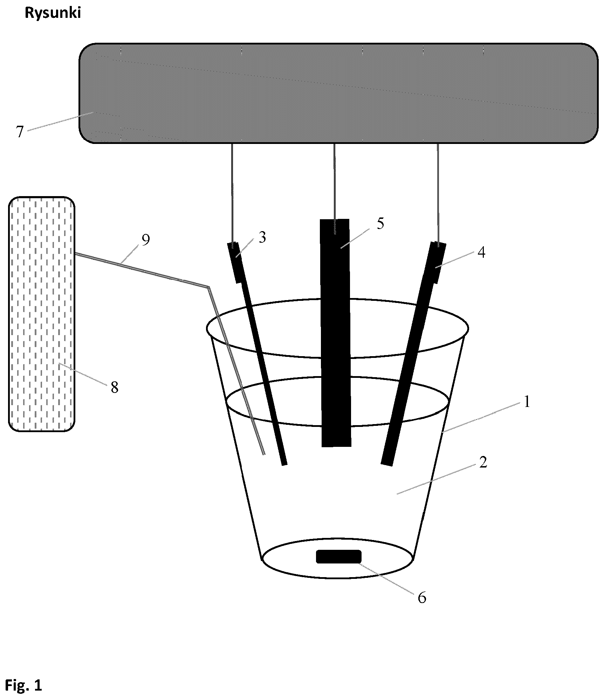

**Fig. 1** shows a diagram of a electrochemical cell;

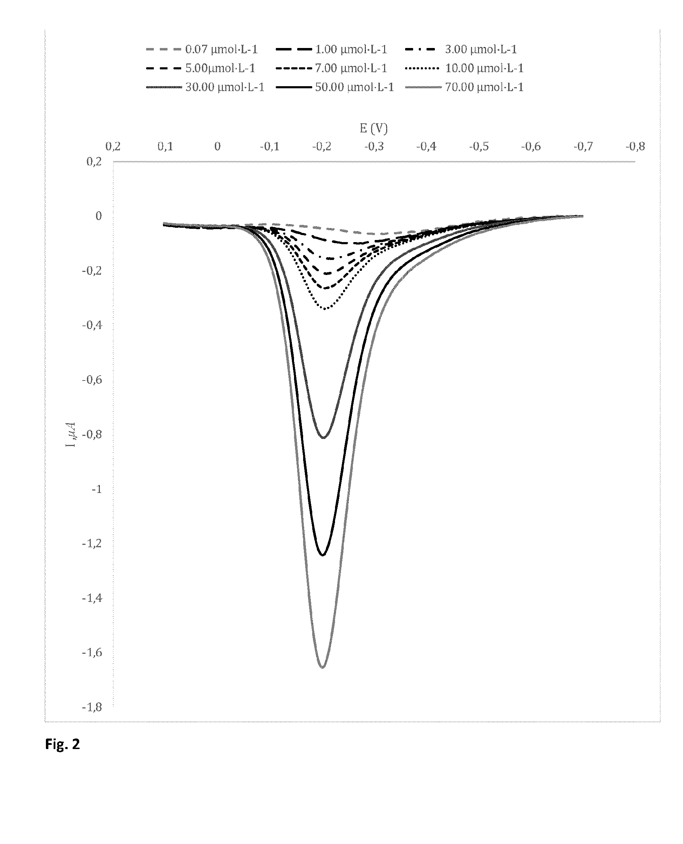

**Fig. 2** shows voltammograms recorded for increasing concentration of the thymoquinone standard solution for example 1;

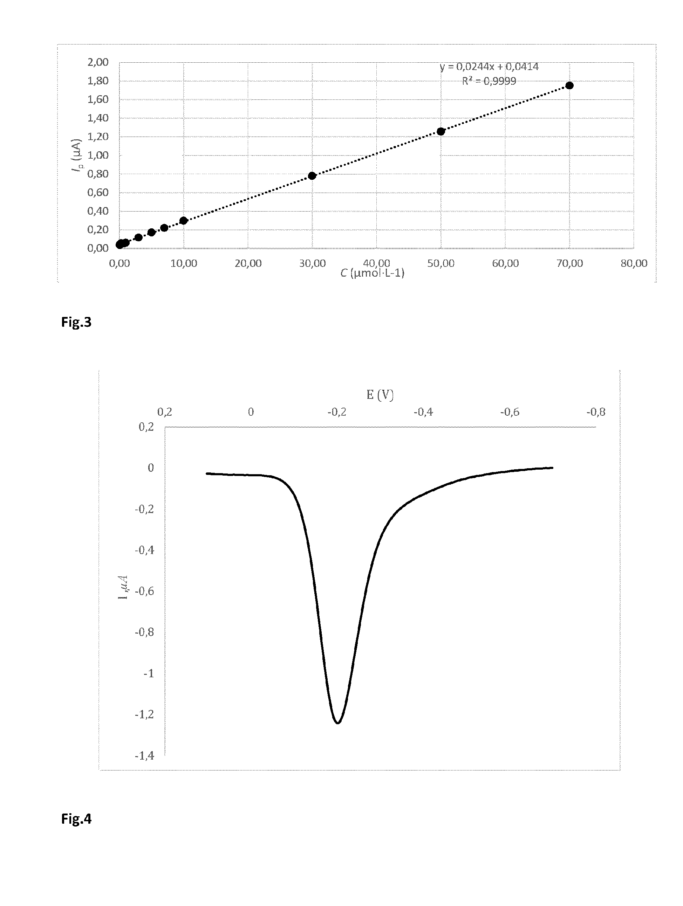

**Fig. 3** shows a calibration curve for example 1;
**Fig. 4** shows the voltammogram obtained for the test sample (discussed in Example 2);

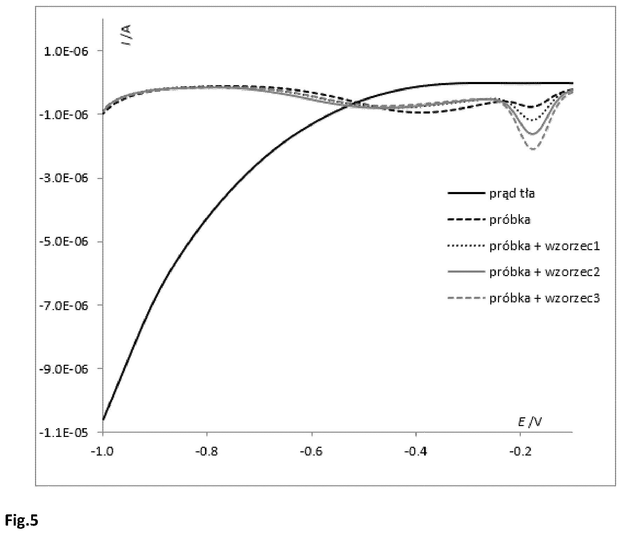

**Fig. 5** shows voltammograms recorded for the blank, for the sample and for increasing concentration of the thymoquinone standard solution, according to example 2;

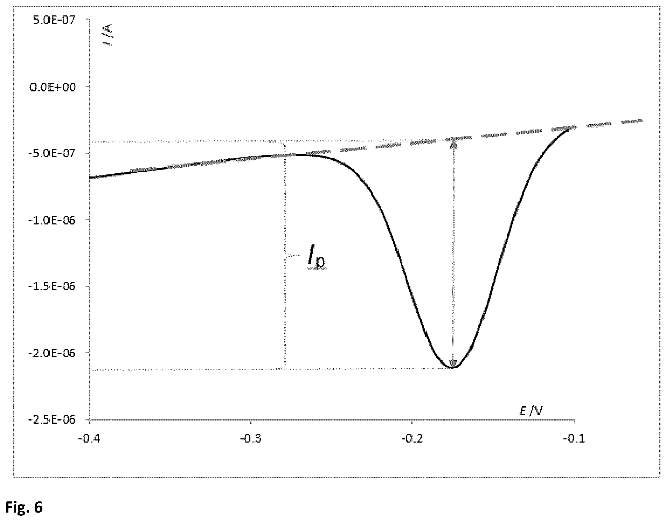

**Fig. 6** shows how the peak current is measured.

### EXAMPLE 1

Determination of thymoquinone in a sample of an aqueous solution.

### 1) Recording a blank

Electrochemical cell 1 with a capacity of 15 ml, made of glass, was placed with 10 ml of the supporting electrolyte solution 2, which is an equimolar solution of acetic acid, boric acid and phosphoric acid at a concentration of 0.04 mol/L, adjusted to pH = 9.0 with 0.2 mol/L sodium hydroxide solution. A platinum electrode 3, a saturated silver chloride electrode 4, a boron-doped diamond electrode 5, and a magnetic stirrer 6 were successively introduced into the electrochemical cell. Measurements were carried out using a potentiostat 7, which allows operation in three-electrode systems. All three electrodes were connected to potentiostat 7, each to a dedicated connection The supporting electrolyte 2 was deoxidized each time before the measurement for five minutes, using argon supplied to the electrochemical cell from the gas cylinder 8, which is the gas source, via tube 9. The solution was stirred during deoxidation. In the solution prepared in this way, using the square wave voltammetry technique, a voltammogram was recorded in the potential range from 0.1V to - 0.7V, which constitutes the blank. The required parameters of the SWV technique are an amplitude of 30 m V, a step potential of 5 V, and a frequency of 25 Hz.

### 2) Preparation of calibration curve for the determination of thymoquinone

To perform the determination according to the method, it is necessary to use a standard solution of thymoquinone with a concentration of 0.001 mol/L, which was prepared by weighing 8.21 mg of the analyte, quantitatively transferring the substance to a 50 ml volumetric flask and filling up to volume with distilled water. The solution was then mixed and set aside for 48 hours. Initially, steps were taken as described in point 1). Then, aliquots of the thymoquinone standard solution were added to the electrochemical cell with the supporting electrolyte so that its concentration in the electrochemical cell ranged from 0.07 µmol/L to 70.0 µmol/L. After each addition of the standard solution, a voltammogram was recorded. Then the values of the peak current were measured. The peak current corresponding to the thymoquinone content is visible at a potential of -220mV.

Three measurement series were performed, obtaining peak currents for thymoquinone concentrations ranging from 0.07 µmol/L to 70.0 µmol/L. The obtained results of the peak currents for individual concentrations were averaged.

From the averaged peak current values the relationship was prepared *I*ₚ = *f*(c_{Tym}), where:
*I*ₚ denotes peak current at a potential of -220mV
c_{Tym} denotes concentration of thymoquinone in the electrochemical cell.
For *I*ₚ = *f*(c_{Tym}), equation of the calibration curve was found.

### 3) Determination of thymoquinone in a water sample

50 µL of the sample was added to the electrochemical cell placed with the supporting electrolyte, then the voltammogram was recorded and the peak current was measured. The peak current corresponding to the thymoquinone content is visible at a potential of -220 mV. The value of the peak current for the sample was within the range of calibration curve. Then, using the equation of the calibration curve prepared as described in point 2), substituting the *I*ₚ value for the value of the peak current of the sample, the concentration of thymoquinone in the electrochemical cell and then in the sample was calculated.

### EXAMPLE 2

Determination of thymoquinone in an oil sample.

### 1) Recording blank

Electrochemical cell 1 with a capacity of 15 ml, made of glass, was placed with 9.5 ml of the supporting electrolyte solution 2, which is an equimolar solution of acetic acid, boric acid and phosphoric acid at a concentration of 0.04 mol/L, adjusted to pH = 10.0 with 0.2 mol/L hydroxide sodium solution. A platinum electrode 3, a silver chloride electrode 4, a amalgam film electrode 5, and a magnetic stirrer 6 were successively introduced into the electrochemical cell. Measurements were carried out using a potentiostat 7, which allows operation in three-electrode systems. All three electrodes were connected to potentiostat 7, each to a dedicated connection. The supporting electrolyte solution 1 was deoxidized each time before the measurement for five minutes, using argon supplied to the electrochemical cell from the gas cylinder 8, which is the gas source, via tube 9. The solution was stirred during deoxidation. In the solution prepared in this way, using the square wave voltammetry technique, a voltammogram was recorded in the potential range from -0.05V to -1.2V, which constitutes the blank. The required parameters of the SWV technique are an amplitude of 25 mV, a step potential of 4 mV, and a frequency of 25 Hz.

### 2) Determination of thymoquinone in real samples (oil)

To determine thymoquinone, it is necessary to prepare a food sample. For this purpose, 5.0 mg of oil was weighed and placed in a 5 ml volumetric flask, filled up to the volume and mixed. The solution obtained in this way is called the sample solution.

To perform the determination according to the method, it is necessary to use a standard solution of thymoquinone with a concentration of 0.001 mol/L, which was prepared by weighing 8.21 mg of the analyte, quantitatively transferring the substance to a 50 ml volumetric flask and filling it up to the volume with methanol. The solution was then mixed. Determining the thymoquinone content in a sample requires recording four voltammograms.

The first voltammogram is the voltammogram of the sample, to be recorded it was placed in an electrochemical cell 1 containing 9.5mL of the supporting electrolyte 2, 0.5mL of the sample solution. After recording the voltammogram, the peak current was measured. The peak current corresponding to the thymoquinone content is visible at a potential of -165 mV. The current value for the sample ranges from 2.8 x 10 ⁻⁷ A to 3.2 x 10 ⁻⁷ A.

The second voltammogram was recorded by adding 1 portion of the standard thymoquinone solution containing 50 nanomoles of thymoquinone to the electrochemical cell. After recording the second voltammogram, the peak current was measured at a potential of -165 mV.

The third and fourth voltammograms were recorded analogously after subsequent additions of the standard thymoquinone solution. The entire sequence of steps described in points 1 and 2 of the example was repeated three times. For each repetition, from the obtained current values, equation was found for the relationship *I*ₚ = *f*(n_{tym wz}), where
*I*ₚ denotes peak current at a potential of -165mV,
n_{tym wz} denotes the amount of moles of thymoquinone added to the electrochemical cell.

Then, using the obtained equations, the content of thymoquinone in the electrochemical cell and then in the sample was calculated. The obtained thymoquinone content results were averaged.

For the analyzed oil sample, thymoquinone content was determined to be 8.13 ± 0.34 (n=3) mg/ml, while the thymoquinone content determined using the reference method for the same sample was 8.20 ± 0.45 (n=3).

## Claims

1. A method for determining thymoquinone by square wave voltammetry (SWV), **characterized in that** it includes steps where
- in the first step, equal volumes of acetic acid, boric acid and phosphoric acid are mixed at a concentration of 0.04 mol/L and adjusted to pH in the range of 9.0-10.0 to prepare a buffer solution, which is the supporting electrolyte (2);
- in the second step, a sample solution is prepared for analysis
• for samples in which the medium (M) is oil - a solution of a food sample of the tested oil is prepared by dissolving the oil in ethanol in a volumetric flask, then filled up to volume with ethanol and mixed
• for samples in which the medium (M) is water or a water-soluble solvent - the sample solution is prepared by dissolving thymoquinone in water;
- in the third step, the electrochemical cell (1) is placed with the supporting electrolyte (2) and a magnetic stirrer (6), a platinum electrode (3) as an auxiliary electrode, a saturated silver chloride electrode (4) as a reference electrode and a working electrode (5) are introduced successively, where the electrode reaction of thymoquinone reduction takes place at the working electrode surface (5);
- in the fourth step, all three electrodes were connected to the potentiostat (7), each to a dedicated connection, and then the supporting electrolyte (2) is deoxidized for five minutes before measurement, using argon from the gas cylinder (8) constituting the gas source connected to electrochemical cell (1) with a supply tube (9), and the solution is mixed during deoxidation using a magnetic stirrer (6);
- in the fifth step, voltammograms are recorded with the determination of current reference values for standard solutions of known thymoquinone concentrations, and
• for samples in which the medium (M) is oil, the recording of voltammograms includes steps where:
a) voltammogram is recorded for pure supporting electrolyte, without any additives, constituting a blank;
b) the voltammogram of the sample, prepared in the second step, is recorded and the peak current is measured at a potential of -165mV;
c) a standard thymoquinone solution, which is a solution of thymoquinone in methanol, is added to the electrochemical cell (1) and the peak current is measured at a potential of -165 mV;
d) step c) is repeated twice using additions of the standard thymoquinone solution containing the same amount of thymoquinone as added in step c);
e) from the peak current values obtained in points b)-d) the curve *I*ₚ = f(X_{tym}), is plotted, where:
*I*ₚ denotes peak current at a potential of -165 mV and
*X*_{tym} denotes the amount of moles or grams of added thymoquinone;
f) the point of intersection of the obtained curve of step e) is read , and based on the obtained *X*_{tym} , the content of thymoquinone in the electrochemical cell (1) and the sample is calculated;
g) then steps a)-f) are repeated three times and, based on the results obtained, the average content of thymoquinone in the sample is calculated;
, wherein all voltammograms are recorded in the potential range from -0.05V to -1.2V, with the SWV technique parameters being: amplitude 25 mV, step potential 4 mV and frequency 25 Hz;
• for samples in which the medium (M) is water or a water-soluble solvent, recording of voltammograms includes steps where:
h) voltammogram is recorded for pure supporting electrolyte, without any additives, constituting the blank;
i) a standard thymoquinone solution, which is a solution of thymoquinone in water, is added to the electrochemical cell (1) and the peak current is measured at a potential of -220mV ;
j) then step i) is repeated minimum five times, recording voltammograms for the increasing concentration of thymoquinone in the electrochemical cell, which is created by the addition of the standard thymoquinone solution, and each time the peak current is measured at a potential of -220mV;
k) steps h)-j) are repeated three times to obtain three measurement series ;
l) then an relationship *I*ₚ *= f*(c_{Tym}), is determined where:
*I*ₚ denotes averaged value of the current increment at a potential of -220mV from three measurement series,
c_{Tym} denotes thymoquinone concentration in the electrochemical cell;
and equation of calibration curve is determined;
m) then the voltammogram of the sample prepared in the second step is recorded and the peak current is measured at a potential of -220mV;
n) the value of the peak current obtained in step m) is substituted into the equation from step I) as *I*ₚ, calculating the concentration of thymoquinone in the electrochemical cell and then the content in the sample;
o) then steps m)-n) are repeated twice and, based on the results obtained, the average content of thymoquinone in the sample is calculated;
, wherein all voltammograms are recorded in the potential range from 0.1V to -0.7V, with the SWV technique parameters being: amplitude 30mV, step potential 5mV and frequency 25Hz.

2. The method according to claim 1, **characterized in that** the pH of the supporting electrolyte is adjusted to the appropriate pH using sodium or potassium hydroxide solutions.

3. Method according to claim 2, **characterized in that** in the first step the pH of the supporting electrolyte is adjusted to pH equal to 9.0 with 0.2M sodium hydroxide, where in the second step the working electrode (5) is a boron doped diamond electrode, and the medium (M) is water or a water-soluble solvent.

4. Method according to claim 2, **characterized in that** in the first step the pH of the supporting electrolyte is adjusted to pH equal to 10.0 with 0.2M potassium hydroxide, where in the second step the working electrode (5) is an amalgam film electrode, with the medium (M) being oil.

## Patentansprüche

1. Verfahren zur Bestimmung von Thymochinon durch Rechteckwellen Voltammetrie (SWV), **dadurch gekennzeichnet, dass** es Schritte umfasst, wobei
- Im ersten Schritt gleiche Mengen Essigsäure, Borsäure und Phosphorsäure in einer Konzentration von 0,04 mol/l gemischt und auf einen pH-Wert im Bereich von 9,0-10,0 eingestellt werden, um eine Pufferlösung herzustellen, die als Leitelektrolyte dient (2);
- Im zweiten Schritt eine Probenlösung zur Analyse vorbereitet wird
• für Proben, bei denen das Medium (M) Öl ist - eine Lösung einer Lebensmittelprobe des getesteten Öls wird hergestellt, indem das Öl in Ethanol in einem Messkolben gelöst, dann mit Ethanol aufgefüllt und gemischt wird
• für Proben, bei denen das Medium (M) Wasser oder ein wasserlösliches Lösungsmittel ist - die Probenlösung wird durch Auflösen von Thymochinon in Wasser hergestellt;
- Im dritten Schritt wird die elektrochemische Zelle (1) mit dem Leitelektrolyten (2) und einem Magnetrührer (6) versehen, eine Platinelektrode (3) als Hilfselektrode, eine gesättigte Silberchloridelektrode (4) als Referenzelektrode und eine Arbeitselektrode (5) werden nacheinander eingeführt, wobei an der Oberfläche der Arbeitselektrode (5) die Elektrodenreaktion der Thymochinon-Reduktion stattfindet;
- Im vierten Schritt wurden alle drei Elektroden mit dem Potentiostaten (7) verbunden, jede mit einem eigenen Anschluss, und dann wurde der Leitelektrolyt (2) vor der Messung fünf Minuten lang mit Argon aus der Gasflasche (8) desoxidiert, die die Gasquelle darstellt und über ein Versorgungsrohr (9) mit der elektrochemischen Zelle (1) verbunden ist, und die Lösung wurde während der Desoxidation mit einem Magnetrührer (6) gemischt;
- Im fünften Schritt werden Voltammogramme mit der Bestimmung aktueller Referenzwerte für Standardlösungen bekannter Thymochinon-Konzentrationen aufgezeichnet und
• Bei Proben, bei denen das Medium (M) Öl ist, umfasst die Aufzeichnung von Voltammogrammen folgende Schritte:
a) Das Voltammogramm wird für einen reinen Leitelektrolyten ohne jegliche Zugaben aufgezeichnet, der eine Blindprobe darstellt;
b) Das Voltammogramm der im zweiten Schritt hergestellten Probe wird aufgezeichnet und der Spitzenstrom bei einem Potential von -165 mV gemessen;
c) Eine Standard-Thymochinon-Lösung, d. h. eine Lösung von Thymochinon in Methanol, wird der elektrochemischen Zelle (1) hinzugefügt und der Spitzenstrom bei einem Potential von -165 mV gemessen;
d) Schritt c) wird zweimal wiederholt, wobei Zugaben der Standard-Thymochinon-Lösung verwendet werden, die die gleiche Menge Thymochinon enthalten, wie in Schritt c) hinzugefügt;
e) Aus den in den Punkten b) - d) ermittelten Spitzenstromwerten wird die Kurve *I*ₚ = f (X_{tym}) erstellt, wobei:
*I*ₚ den Spitzenstrom bei einem Potential von -165 mV bezeichnet und
X _{tym} die Menge an Mol oder Gramm des hinzugefügten Thymochinons
bezeichnet;
f) Der Schnittpunkt der in Schritt e) erhaltenen Kurve wird abgelesen und basierend auf dem erhaltenen Xtym wird der Thymochinon-Gehalt in der elektrochemischen Zelle (1) und der Probe berechnet;
g) Anschließend werden die Schritte a) - f) dreimal wiederholt und anhand der erhaltenen Ergebnisse der durchschnittliche Thymochinon-Gehalt der Probe berechnet;
wobei alle Voltammogramme im Potentialbereich von -0,05 V bis -1,2 V aufgezeichnet werden, wobei die Parameter der SWV-Technik wie folgt sind: Amplitude 25 mV, Sprungpotential 4 mV und Frequenz 25 Hz;
• Bei Proben, bei denen das Medium (M) Wasser oder ein wasserlösliches Lösungsmittel ist, umfasst die Aufzeichnung von Voltammogrammen folgende Schritte:
h) Das Voltammogramm wird für den reinen Leitelektrolyten ohne jegliche Zusätze aufgezeichnet, der die Blindprobe bildet.
i) Eine Standard-Thymochinon-Lösung, d. h. eine Lösung von Thymochinon in Wasser, wird der elektrochemischen Zelle (1) hinzugefügt und der Spitzenstrom bei einem Potential von -220 mV gemessen;
j) Dann wird Schritt i) mindestens fünfmal wiederholt, wobei Voltammogramme für die zunehmende Thymochinon-Konzentration in der elektrochemischen Zelle aufgezeichnet werden, die durch die Zugabe der Standard-Thymochinon-Lösung entsteht, und jedes Mal wird der Spitzenstrom bei einem Potential von -220 mV gemessen;
k) die Schritte h) - j) werden dreimal wiederholt, um drei Messreihen zu erhalten;
I) dann wird eine Beziehung *I*ₚ = *f*(c_{Tym}) bestimmt, wobei:
*I*ₚ bezeichnet den gemittelten Wert der Stromerhöhung bei einem Potential von - 220 mV aus drei Messreihen,
c_{Tym} bezeichnet die Thymochinon-Konzentration in der elektrochemischen Zelle; und die Gleichung der Kalibrierungskurve wird bestimmt;
m) anschließend wird das Voltammogramm der im zweiten Schritt hergestellten Probe aufgezeichnet und der Spitzenstrom bei einem Potential von -220 mV gemessen;
n) der in Schritt m) erhaltene Wert des Spitzenstroms wird als *I*ₚ in die Gleichung aus Schritt I) eingesetzt, wodurch die Thymochinon-Konzentration in der elektrochemischen Zelle und dann der Gehalt in der Probe berechnet werden;
o) anschließend werden die Schritte m) - n) zweimal wiederholt und anhand der erhaltenen Ergebnisse der durchschnittliche Thymochinon-Gehalt der Probe berechnet;
wobei alle Voltammogramme im Potentialbereich von 0,1 V bis -0,7 V aufgezeichnet werden, dabei sind die Parameter der SWV-Technik wie folgtsind: Amplitude 30 mV, Stufenpotential 5 mV und Frequenz 25 Hz.

2. Verfahren nach Anspruch 1, **dadurch gekennzeichnet, dass** der pH-Wert des Leitelektrolyten mit Natrium- oder Kaliumhydroxid-Lösungen auf den entsprechenden pH-Wert eingestellt wird.

3. Verfahren nach Anspruch 2, **dadurch gekennzeichnet, dass** im ersten Schritt der pH-Wert des Leitelektrolyten mit 0,2 M Natriumhydroxid auf einen pH-Wert von 9,0 eingestellt wird, wobei im zweiten Schritt die Arbeitselektrode (5) eine mit Bor dotierte Diamantelektrode ist und das Medium (M) Wasser oder ein wasserlösliches Lösungsmittel ist.

4. Verfahren nach Anspruch 2, **dadurch gekennzeichnet, dass** im ersten Schritt der pH-Wert des Leitelektrolyten mit 0,2 M Kaliumhydroxid auf einen pH-Wert von 10,0 eingestellt wird, wobei im zweiten Schritt die Arbeitselektrode (5) eine Amalgamfilmelektrode ist und das Medium (M) Öl ist.

## Revendications

1. Procédé de détermination de la thymoquinone par voltamétrie à onde carrée (SWV), **caractérisé en ce qu'il** comprend les étapes où
- dans la première étape, des volumes égaux d'acide acétique, d'acide borique et d'acide phosphorique sont mélangés à une concentration de 0,04 mol/L et ajustés à un pH 9,0 - 10,0 pour préparer une solution tampon, qui est l'électrolyte de support (2) ;
- dans la deuxième étape, une solution d'échantillon est préparée pour analyse
• pour les échantillons dans lesquels le milieu (M) est l'huile - une solution d'un échantillon alimentaire de l'huile testée est préparée en dissolvant l'huile dans de l'éthanol dans une fiole jaugée, puis complétée au volume avec de l'éthanol et mélangée
• pour les échantillons dans lesquels le milieu (M) est l'eau ou un solvant hydrosoluble - la solution d'échantillon est préparée en dissolvant la thymoquinone dans l'eau ;
- dans la troisième étape, la cellule électrochimique (1) est placée avec l'électrolyte de support (2) et un agitateur magnétique (6), une électrode de platine (3) comme électrode auxiliaire, une électrode de chlorure d'argent saturé (4) comme électrode de référence et une électrode de travail (5) sont introduites successivement, où la réaction d'électrode de réduction de la thymoquinone a lieu à la surface de l'électrode de travail (5) ;
- dans la quatrième étape, les trois électrodes ont été connectées au potentiostat (7), chacune à une connexion dédiée, puis l'électrolyte de support (2) est désoxydé pendant cinq minutes avant la mesure, en utilisant de l'argon provenant de la bouteille de gaz (8) constituant la source de gaz connectée à la cellule électrochimique (1) avec un tube d'alimentation (9), et la solution est mélangée pendant la désoxydation à l'aide d'un agitateur magnétique (6) ;
- dans la cinquième étape, des voltamogrammes sont enregistrés avec la détermination des valeurs de référence actuelles pour des solutions standard de concentrations connues en thymoquinone, et
• pour les échantillons dont le milieu (M) est l'huile, l'enregistrement des voltampérogrammes comprend les étapes où :
a) le voltamogramme est enregistré pour un électrolyte de support pur, sans aucun additif, constituant un blanc ;
b) le voltamogramme de l'échantillon, préparé à la deuxième étape, est enregistré et le courant de crête est mesuré à un potentiel de -165 mV ;
c) une solution standard de thymoquinone, qui est une solution de thymoquinone dans du méthanol, est ajoutée à la cellule électrochimique (1) et le courant de crête est mesuré à un potentiel de -165 mV ;
d) l'étape c) est répétée deux fois en utilisant des ajouts de la solution standard de thymoquinone contenant la même quantité de thymoquinone que celle ajoutée à l'étape c) ;
e) à partir des valeurs de courant de crête obtenues aux points b)-d) la courbe *Iₚ* = f (X_{tym}), est tracée, où :
*Iₚ* désigne le courant de crête à un potentiel de -165 mV et
*X*_{tym} désigne la quantité de moles ou de grammes de thymoquinone ajoutée ;
f) le point d'intersection de la courbe obtenue de l'étape e) est lu et, sur la base du *X*_{tym} obtenu, la teneur en thymoquinone dans la cellule électrochimique (1) et l'échantillon est calculée ;
g) puis les étapes a) à f) sont répétées trois fois et, sur la base des résultats obtenus, la teneur moyenne en thymoquinone dans l'échantillon est calculée ;
, dans lequel tous les voltampérogrammes sont enregistrés dans la plage de potentiel de - 0,05 V à -1,2 V, les paramètres de la technique SWV étant : amplitude 25 mV, potentiel de pas 4 mV et fréquence 25 Hz ;
• pour les échantillons dans lesquels le milieu (M) est l'eau ou un solvant hydrosoluble, l'enregistrement des voltampérogrammes comprend les étapes où :
h) le voltamogramme est enregistré pour l'électrolyte de support pur, sans aucun additif, constituant le blanc ;
i) une solution standard de thymoquinone, qui est une solution de thymoquinone dans l'eau, est ajoutée à la cellule électrochimique (1) et le courant de crête est mesuré à un potentiel de -220 mV ;
j) puis l'étape i) est répétée au moins cinq fois, en enregistrant des voltampérogrammes pour la concentration croissante de thymoquinone dans la cellule électrochimique, qui est créée par l'ajout de la solution standard de thymoquinone, et à chaque fois le courant de crête est mesuré à un potentiel de -220 mV ;
k) les étapes h) à j) sont répétées trois fois pour obtenir trois séries de mesures ;
l) alors une relation *I*ₚ = *f*(c_{Tym}), est déterminée où :
*Iₚ* désigne la valeur moyenne de l'incrément de courant à un potentiel de -220 mV à partir de trois séries de mesures,
c_{Tym} désigne la concentration en thymoquinone dans la cellule électrochimique ; et l'équation de la courbe d'étalonnage est déterminée ;
m) ensuite, le voltamogramme de l'échantillon préparé à la deuxième étape est enregistré et le courant de crête est mesuré à un potentiel de -220 mV ;
n) la valeur du courant de crête obtenue à l'étape m) est substituée dans l'équation de l'étape I) par *I*ₚ, en calculant la concentration de thymoquinone dans la cellule électrochimique puis la teneur dans l'échantillon ;
o) puis les étapes m) à n) sont répétées deux fois et, sur la base des résultats obtenus, la teneur moyenne en thymoquinone dans l'échantillon est calculée ;
, dans lequel tous les voltampérogrammes sont enregistrés dans la plage de potentiel de 0,1 V à -0,7 V, les paramètres de la technique SWV étant : amplitude 30 mV, potentiel de pas 5 mV et fréquence 25 Hz.

2. Procédé selon la revendication 1, **caractérisé en ce que** le pH de l'électrolyte de support est ajusté au pH approprié à l'aide de solutions d'hydroxyde de sodium ou de potassium.

3. Procédé selon la revendication 2, **caractérisé en ce que** dans la première étape le pH de l'électrolyte de support est ajusté à un pH égal à 9,0 avec de l'hydroxyde de sodium 0,2 M, où dans la deuxième étape l'électrode de travail (5) est une électrode de diamant dopée au bore, et le milieu (M) est de l'eau ou un solvant hydrosoluble.

4. Procédé selon la revendication 2, **caractérisé en ce que** dans la première étape le pH de l'électrolyte de support est ajusté à un pH égal à 10,0 avec de l'hydroxyde de potassium 0,2 M, où dans la deuxième étape l'électrode de travail (5) est une électrode à film d'amalgame, le milieu (M) étant de l'huile.
